# FASCICULE DE BREVET EUROPEEN

(11) **EP 4 532 680 B1**
(45) Date de publication et mention de la délivrance du brevet: **18.03.2026**
(21) Numéro de dépôt: 23727583.9
(22) Date de dépôt: 22.05.2023
(51) Int. Cl.: C12M 3/06, B01L 3/00, C12M 1/00

(54) **DISPOSITIF DE CAPTURE D'UN OBJET BIOLOGIQUE, PROCÉDÉ ET SYSTÈME D'ANALYSE D'UN OBJET BIOLOGIQUE UTILISANT LEDIT DISPOSITIF DE CAPTURE**
VORRICHTUNG ZUM ERFASSEN EINES BIOLOGISCHEN OBJEKTS SOWIE VERFAHREN UND SYSTEM ZUM ANALYSIEREN EINES BIOLOGISCHEN OBJEKTS MIT DER ERFASSUNGSVORRICHTUNG
DEVICE FOR CAPTURING A BIOLOGICAL OBJECT, AND METHOD AND SYSTEM FOR ANALYSING A BIOLOGICAL OBJECT USING THE CAPTURING DEVICE

(30) Priorité: 25.05.2022 FR 2205064
(43) Date de publication de la demande: 09.04.2025
(73) Titulaire: Commissariat à l'Energie Atomique et aux Energies Alternatives, 75015 Paris (FR)
(72) Inventeur: AGACHE, Vincent, 38054 Grenoble cedex 09 (FR)
(74) Mandataire: INNOV-GROUP
(86) Numéro de dépôt international: PCT/EP2023/063555
(87) Numéro de publication internationale: WO 2023/227493

(56) Documents cités:
- FR-A1- 3 107 901
- US-A1- 2021 039 104
- TANG WENLAI ET AL: "A portable single-cell analysis system integrating hydrodynamic trapping with broadband impedance spectroscopy", SCIENCE CHINA TECHNOLOGICAL SCIENCES, SCIENCE CHINA PRESS, HEIDELBERG, vol. 60, no. 11, 18 October 2017 (2017-10-18), pages 1707 - 1715, XP036407337, ISSN: 1674-7321, [retrieved on 20171018], DOI: 10.1007/S11431-017-9129-7

## Description

### Domaine technique de l'invention

La présente invention se rapporte à un dispositif de capture d'un objet biologique, à un procédé et un système d'analyse d'un objet biologique utilisant ledit dispositif de capture.

### Etat de la technique

Il est connu de vouloir isoler un objet biologique dans un circuit fluidique pour étudier ses sécrétions et ses réactions à différentes substances. C'est par exemple le cas pour des cellules tumorales qui vont être amenées à secréter des vésicules extracellulaires, tels que des exosomes. Pour isoler l'objet biologique, il est connu d'utiliser un piège hydrodynamique dans lequel l'objet biologique vient se piéger pendant toute la durée de l'analyse. Ce type de piège hydrodynamique est notamment décrit dans les publications référencées ci-dessous :
- Wei-Heong Tan and Shoji Takeuchi, A trap-and-release integrated microfluidic system for dynamic microarray applications, January 23, 2007, 104(4)1146-1151, PN*AS.*
- RJ Kimmerling, GL Szeto, JW Li, AS Genshaft, SW Kazer, KP Payer, JD Borrajo, JC Blainey, DJ Irvine, AK Shalek, SR Manalis. A microfluidic platform enabling single cell RNA-seq of multigenerational lineages, Nature Communications, 7, Article number: 10220, (2016*).*

Cependant, selon les manipulations effectuées, il est fréquent de créer des différentiels de pression dans le circuit fluidique, susceptibles d'inverser le sens d'écoulement dans le circuit fluidique. Cela peut se présenter lorsque l'opérateur est amené à changer les milieux à injecter dans le circuit fluidique ou tout simplement lorsque les pressions entre les différents accès fluidiques sont déséquilibrées. Dans cette situation, l'objet biologique peut être amené à s'échapper de son piège hydrodynamique, perturbant ainsi l'analyse.

La demande de brevet US2021/039104A1 décrit un système de piégeage de cellules, utilisant plusieurs pièges. Il permet de piéger les cellules lorsque celles-ci sont injectées dans le circuit fluidique dans un sens ou dans le sens opposé. Sa structure ne permet cependant pas de maintenir un objet biologique dans son circuit fluidique, en cas d'inversion du sens d'écoulement dans le circuit fluidique.

Le but de l'invention est de proposer une solution permettant d'assurer un maintien d'un objet biologique dans le circuit fluidique, même en cas d'inversion du sens d'écoulement dans le circuit fluidique.

### Exposé de l'invention

Ce but est atteint par un dispositif de capture d'un objet biologique, comprenant un premier circuit fluidique, ledit premier circuit fluidique comportant :
- Un premier accès fluidique et un deuxième accès fluidique,
- Un canal principal s'étendant entre le premier accès fluidique et le deuxième accès fluidique, comprenant une première branche dans laquelle débouche le premier accès fluidique, une branche centrale prolongeant ladite première branche, via une première branche de jonction, et une deuxième branche prolongeant ladite branche centrale via une deuxième branche de jonction et débouchant sur le deuxième accès fluidique,
- Un premier piège hydrodynamique et un deuxième piège hydrodynamique,
- Chaque piège hydrodynamique étant réalisé sous la forme d'une branche de dérivation par rapport au canal principal et comportant un logement dimensionné pour accueillir ledit objet biologique et une restriction prolongeant ledit logement,
- La première branche et la deuxième branche du canal principal étant dénuées de logement appartenant à un piège hydrodynamique,
- Le premier piège hydrodynamique étant agencé de sorte que son logement communique d'un côté avec la branche centrale du canal principal et que sa restriction débouche de l'autre côté dans la première branche du canal principal,
- Le deuxième piège hydrodynamique étant agencé de sorte que son logement communique d'un côté avec la branche centrale du canal principal et que sa restriction débouche de l'autre côté dans la deuxième branche du canal principal.

Contrairement à l'enseignement de la demande de brevet US2021/039104A1**,** on note que le dispositif de capture de l'invention est dénué de piège hydrodynamique sur la première branche et la deuxième branche de son canal principal. Dans le dispositif décrit dans ce document antérieur, ces deux branches disposent de logements pour venir capturer les cellules, ce qui ne permet pas à un tel dispositif de remplir l'objectif de maintenir l'objet biologique dans le circuit fluidique. En effet, lorsque les cellules sont injectées dans le circuit suivant un premier sens découlement ("forward flow" par exemple dans le document D1), celles-ci viennent se loger dans les premiers logements accessibles sur la première branche. En cas d'inversion du sens d'écoulement ("reverse flow"), les cellules peuvent s'échapper de ces premiers logements et ne sont donc pas conservées dans le circuit fluidique. La différence structurelle entre l'invention et le dispositif antérieur permet donc d'obtenir l'effet technique de maintien de l'objet biologique dans le circuit fluidique, même en cas d'inversion des flux.

L'invention vise également un système d'analyse utilisant ledit dispositif de capture, le dispositif de capture garantissant le maintien de l'objet biologique dans le circuit fluidique pendant toute la durée de l'analyse, même en cas d'inversion du sens d'écoulement dans le circuit fluidique.

Le système d'analyse d'un objet biologique comporte un dispositif de capture dudit objet biologique et un dispositif de mesure :
- Le dispositif de capture étant tel que ci-dessus,
- Le dispositif de mesure comportant :
   ∘ Une branche fluidique de mesure connectée à une première extrémité à la branche centrale du dispositif de capture, entre ses deux pièges hydrodynamiques, et à une deuxième extrémité à un deuxième circuit fluidique, distinct du premier circuit fluidique,
   ∘ Un capteur coopérant avec la branche fluidique de mesure.

Selon une particularité, le deuxième circuit fluidique comporte une branche fluidique de récupération s'étendant entre un troisième accès fluidique et un quatrième accès fluidique du deuxième circuit fluidique, ladite deuxième extrémité de la branche fluidique de mesure étant connectée sur la branche fluidique de récupération, entre le troisième accès fluidique et le quatrième accès fluidique.

Selon une autre particularité, le capteur est de type résonateur, de type photonique ou est configuré pour mesurer une grandeur électrique à travers la branche fluidique de mesure.

Selon une autre particularité, le premier circuit fluidique et le deuxième circuit fluidique sont réalisés dans un composant fabriqué par micro-fabrication.

L'invention vise enfin un procédé d'analyse mis en œuvre à l'aide dudit système d'analyse.

Le procédé d'analyse d'un objet biologique est mis en œuvre à l'aide du système d'analyse tel que défini ci-dessus, le procédé comportant :
- Une première étape d'injection de l'objet biologique par le premier accès fluidique par application d'une première valeur de pression sur le premier accès fluidique, supérieure à une deuxième valeur de pression présente sur le deuxième accès fluidique,
- Piégeage hydrodynamique de l'objet biologique dans le deuxième piège hydrodynamique,
- Application d'une troisième valeur de pression sur le premier accès fluidique et le deuxième accès fluidique, et application d'une quatrième valeur de pression sur le troisième accès fluidique supérieure à une cinquième valeur de pression présente sur le quatrième accès fluidique et inférieure à ladite troisième valeur de pression,
- Récupération des sécrétions générés par ledit objet biologique au niveau du quatrième accès fluidique, après passage à travers la branche fluidique de mesure.

### Brève description des figures

D'autres caractéristiques et avantages vont apparaître dans la description détaillée qui suit faite en regard des dessins annexés dans lesquels :
- La figure 1 représente un dispositif de capture d'un objet biologique conforme à l'invention ;
- Les figures 2A à 2E illustrent le principe de fonctionnement du dispositif de capture de la figure 1 ;
- La figure 3 représente un système d'analyse d'un objet biologique conforme à l'invention, intégrant ledit dispositif de capture de la figure 1 ;
- Les figures 4A à 4C illustrent les différentes étapes de mise en œuvre d'un procédé d'analyse d'un objet biologique, selon l'invention ;

### Description détaillée d'au moins un mode de réalisation

Dans la suite de la description, on entend par branche fluidique un unique canal fluidique à uniquement deux extrémités dans lequel peut circuler un fluide, chaque extrémité formant un accès fluidique distinct audit canal.

Dans la suite de la description, par objet biologique, on entend par exemple une cellule, un agrégat de cellules, un virus, une bactérie ou autres. Par agrégat de cellules, on entend l'auto-assemblage d'un ou plusieurs types de cellules en trois dimensions. Un tel agrégat de cellules peut notamment s'appeler sphéroïde, organoïde, neuro-sphère. De manière non limitative, l'objet biologique peut par exemple présenter un diamètre allant d'une centaine de nm à quelques µm.

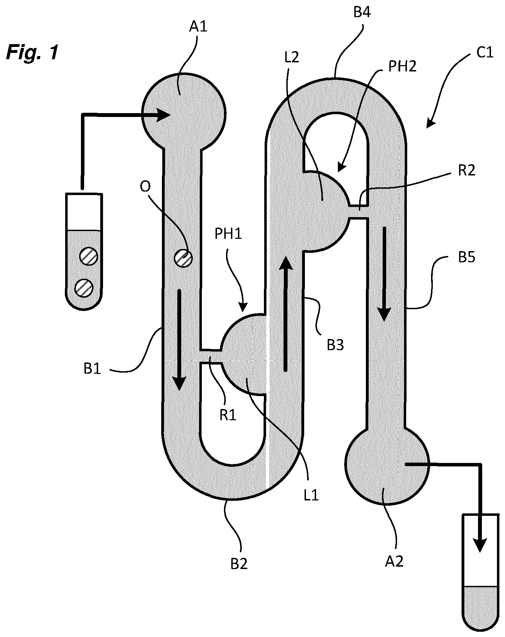

La figure 1 représente un dispositif de capture d'un objet biologique O.

Ce dispositif comporte notamment un réseau fluidique, avantageusement réalisé dans un composant fluidique. Le réseau fluidique comporte des branches fluidiques formées de canaux intégrés audit composant. Ces canaux peuvent être réalisés par exemple par usinage ou à l'aide de toute autre méthode telle que la micro-fabrication. Le composant peut être réalisé à base de verre et/ou de silicium.

De manière plus concrète, le dispositif de capture comporte un premier circuit fluidique C1.

Le premier circuit fluidique C1 comporte un premier accès fluidique A1 et un deuxième accès fluidique A2. Par accès fluidique, on entend une entrée ou une sortie fluidique, par laquelle il est possible d'injecter un fluide ou de récupérer un fluide. Chaque accès fluidique peut avoir le rôle d'entrée fluidique ou de sortie fluidique, selon le sens d'écoulement du fluide dans le circuit fluidique.

Le premier circuit fluidique C1 comporte un canal principal s'étendant entre le premier accès fluidique A1 et le deuxième accès fluidique A2.

De manière non limitative, le canal principal comporte une première branche B1 dans laquelle débouche le premier accès fluidique A1, une branche centrale B3 prolongeant ladite première branche B1, via une première branche de jonction B2, et une deuxième branche B5 prolongeant ladite branche centrale B3 via une deuxième branche de jonction B4 et débouchant sur le deuxième accès fluidique A2.

A titre d'exemple et de manière non limitative, la première branche B1, la branche centrale B3 et la deuxième branche B5 présentent une forme rectiligne. Bien entendu, elles pourraient présenter une autre forme.

De même, à titre d'exemple et de manière non limitative, la première branche de jonction B2 et la deuxième branche de jonction B4 présentent toutes deux une forme coudée, permettant de faire la jonction respectivement entre la première branche B1 et la branche centrale B3 d'une part et entre la branche centrale B3 et la deuxième branche B5 d'autre part.

Le premier accès fluidique A1, la première branche B1, la première branche de jonction B2, la branche centrale B3, la deuxième branche de jonction B4, la deuxième branche B5 et le deuxième accès fluidique A2 sont dimensionnés pour permettre à l'objet biologique O de circuler et de se déplacer dans le premier circuit fluidique C1, lorsqu'il est placé dans un fluide vecteur.

Le premier circuit fluidique C1 comporte également au moins un premier piège hydrodynamique PH1 et un deuxième piège hydrodynamique PH2.

Dans le cadre de l'invention, un piège hydrodynamique est destiné à pouvoir piéger l'objet biologique O de manière à pouvoir mieux surveiller ses sécrétions et son comportement, par exemple lorsqu'il est soumis à des traitements par différentes substances.

Chaque piège hydrodynamique PH1, PH2 est réalisé sous la forme d'une branche de dérivation par rapport au canal principal.

Un piège hydrodynamique comporte ainsi un logement L1, L2 dimensionné pour accueillir ledit objet biologique O et une restriction R1, R2 prolongeant ledit logement, de manière à former une sorte d'entonnoir.

Selon l'invention, les deux pièges hydrodynamiques PH1, PH2 du dispositif sont agencés en miroir. Autrement dit, le premier piège hydrodynamique PH1 est agencé de sorte que son logement L1 communique d'un côté avec la branche centrale B3 du canal principal et que sa restriction R1 débouche de l'autre côté dans la première branche B1 du canal principal.

A l'inverse, le deuxième piège hydrodynamique PH2 est agencé de sorte que son logement L2 communique d'un côté avec la branche centrale B3 du canal principal et que sa restriction R2 débouche dans la deuxième branche B5 du canal principal.

Grâce à cette configuration, l'objet biologique O est toujours maintenu dans l'un des deux pièges, quel que soit le sens d'écoulement du fluide dans le premier circuit fluidique C1. Il faut noter que la première branche B1 et la deuxième branche B5 sont dénuées de logement permettant d'accueillir un objet biologique O. Autrement dit, l'objet biologique ne peut être piégé que lorsqu'il est situé dans la branche centrale B3 du canal principal, dans l'un ou l'autre des logements L1, L2 des deux pièges hydrodynamiques PH1, PH2. Cette particularité structurelle permet de maintenir l'objet biologique O dans la branche centrale B3 du canal principal, même en cas d'inversion du sens d'écoulement.

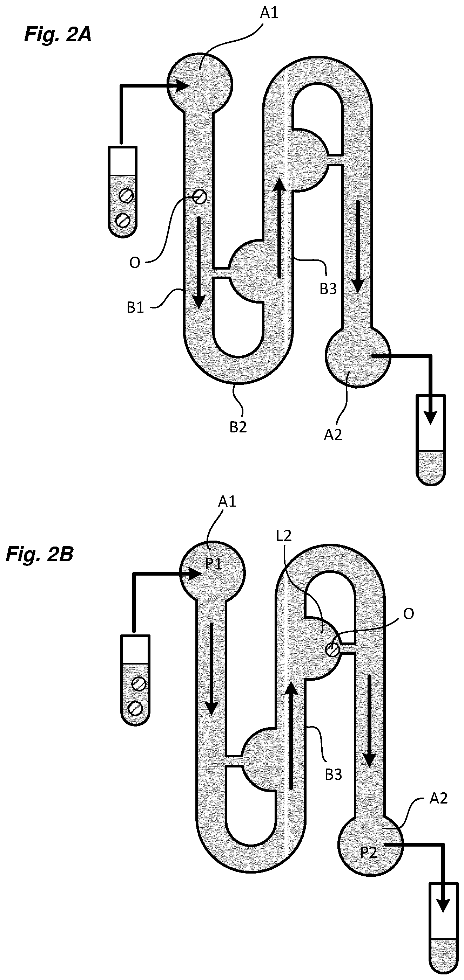

Ce principe est expliqué ci-dessous en liaison avec les figures 2A à 2E.

Figure 2A : On injecte un fluide vecteur comprenant l'objet biologique O à travers le premier accès fluidique A1.

Le fluide vecteur passe ainsi par la première branche B1 puis par la première branche de jonction B2 pour que l'objet biologique O puisse rejoindre la branche centrale B3.

Figure 2B : Compte-tenu du différentiel de pression (P1>P2) entre le premier accès fluidique A1 et le deuxième accès fluidique A2, l'objet biologique O ne peut être capturé dans le premier piège hydrodynamique PH1. L'objet biologique O rencontre ensuite le logement L2 du deuxième piège hydrodynamique PH2 communiquant avec la branche centrale B3 et vient s'y loger.

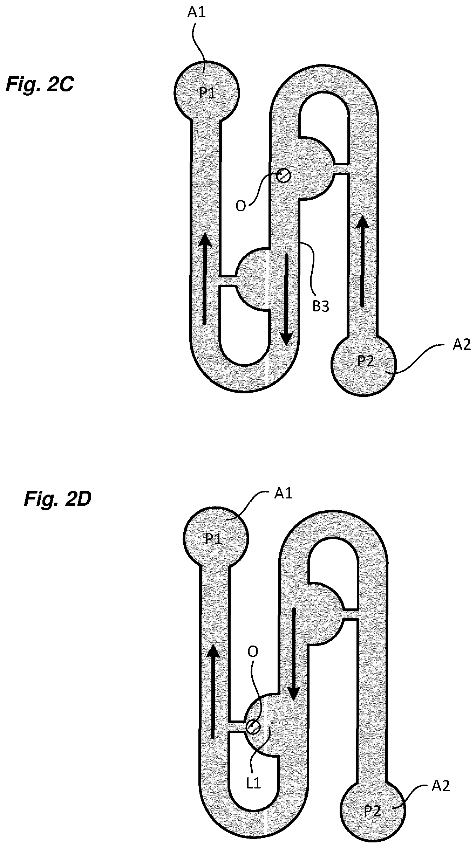

Figure 2C : Par la suite, lorsque le sens d'écoulement dans ce premier circuit fluidique est inversé (par exemple en cas de dépression entre le premier accès fluidique et le deuxième accès fluidique P2>P1), l'objet biologique O est délogé du deuxième piège hydrodynamique PH2 et regagne la branche centrale B3.

Figure 2D : Comme les deux pièges hydrodynamiques PH1, PH2 sont agencés en miroir, l'objet biologique O se dirige vers le premier accès fluidique A1 et rencontre sur son chemin le logement L1 du premier piège hydrodynamique PH1 et vient s'y loger.

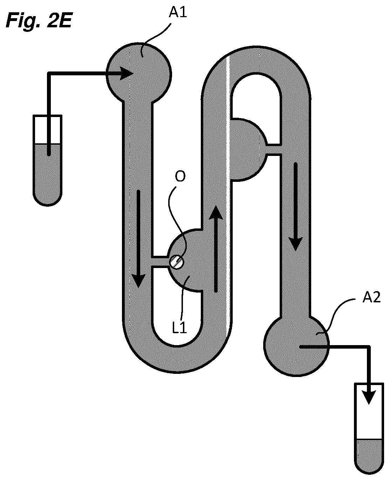

Figure 2E : Après changement du milieu dans le premier circuit fluidique, l'objet biologique O reste piégé dans le logement L1 du premier piège hydrodynamique PH1.

Ainsi lorsque le sens d'écoulement est inversé dans le circuit, l'objet biologique O sort du piège hydrodynamique dans lequel il est, et vient se loger dans le piège symétrique, c'est-à-dire toujours le piège disposé le plus en aval selon le sens d'écoulement du fluide dans le circuit.

Ainsi cette géométrie et configuration en miroir des deux pièges hydrodynamiques PH1, PH2 permet de garantir que l'objet biologique O soit continuellement piégé dans le circuit fluidique, indépendamment du gradient de pression résiduel et des connexions et déconnexions au niveau du premier accès fluidique A1 et du deuxième accès fluidique A2.

Ce dispositif de capture et son principe de fonctionnement sont particulièrement utiles dans un système d'analyse d'un objet biologique.

L'analyse pourra consister par exemple à étudier les sécrétions d'une cellule ou d'un organoïde. Parmi les sécrétions, on peut par exemple citer des exosomes, des vésicules extracellulaires, des particules virales, des protéines, des molécules de type ARN circulant, ADN circulant...

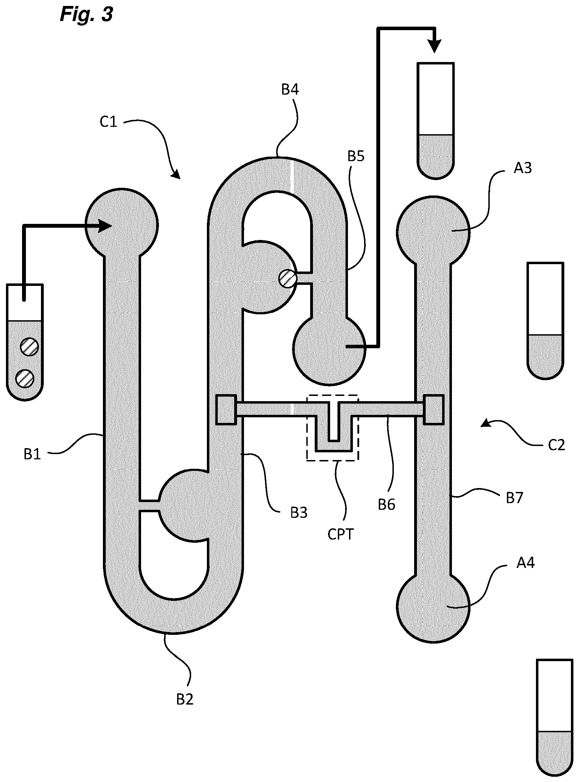

En référence à la figure 3, ce système d'analyse comporte ainsi un dispositif de capture de l'objet biologique O tel que décrit ci-dessus et un dispositif de mesure.

Le dispositif de mesure comporte une branche fluidique de mesure B6 comprenant une première extrémité connectée sur la branche centrale B3 du premier circuit fluidique C1, entre les deux pièges hydrodynamiques PH1, PH2, et une deuxième extrémité connectée sur un deuxième circuit fluidique C2.

La branche fluidique de mesure B6 porte un capteur CPT, configuré pour réaliser des mesures sur le fluide qui traverse la branche fluidique de mesure. Le capteur CPT peut être par exemple positionné sur la branche fluidique de mesure B6 ou traversé par celle-ci. Bien entendu, cela dépendra du type de capteur employé. Il peut notamment s'agir d'un capteur de type résonateur, plus particulièrement appelé capteur SNR pour "Suspended Nanochannel Resonator", qui est positionné pour être traversé par la branche fluidique de mesure B6. Un tel capteur de type SNR est notamment décrit dans la publication intitulée :
*"*Suspended Nanochannel Resonator Arrays with Piezoresistive Sensors for High-Throughput Weighing of Nanoparticles in Solution" - Marco Gagino et al. - ACS Sens 2020, 5, 1230-1238*.*

Bien entendu, il serait possible d'utiliser d'autres types de capteurs. Une solution serait par exemple de mesurer une grandeur électrique (résistance/courant électrique) à travers une particule venant se piéger dans un micropore/nanopore ou le traverser transitoirement, ou une restriction fluidique de façon générale. Un autre exemple serait d'utiliser un capteur photonique de type interféromètre optique (Mach-Zehnder), doté d'une surface fonctionnalisée en fonction de cibles secrétées par l'objet biologique O et destinées à traverser la branche fluidique de mesure B6.

Le deuxième circuit fluidique C2 permet avantageusement de récupérer le fluide contenant les sécrétions générées par l'objet biologique O, après passage à travers la branche fluidique de mesure B6.

Le deuxième circuit fluidique C2 comporte ainsi au moins une branche fluidique B7 de récupération qui s'étend entre un troisième accès fluidique A3 et un quatrième accès fluidique A4.

Il faut noter que chaque accès fluidique du système peut être à une valeur de pression donnée, distincte de celle des autres accès fluidiques.

En fonctionnement, pour récupérer les sécrétions générées par un objet biologique O au niveau du quatrième accès fluidique A4, après passage à travers la branche fluidique de mesure B6, le procédé est le suivant :

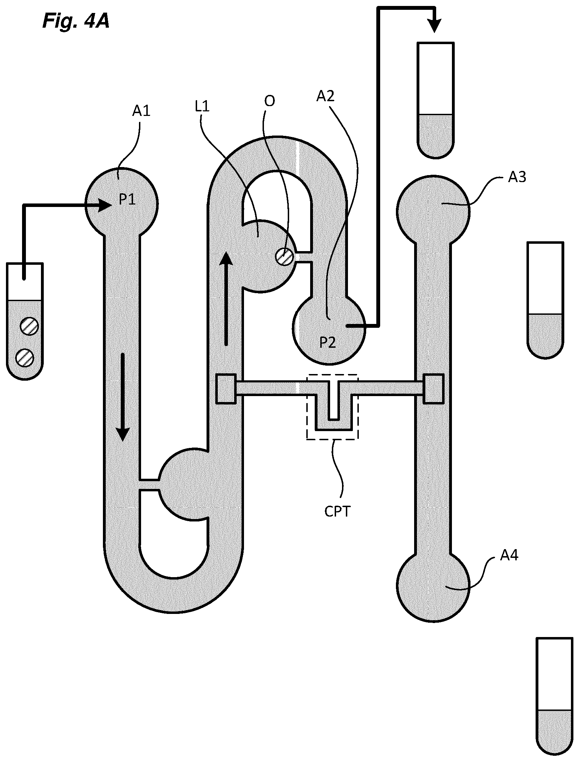

Figure 4A : Après injection via le premier accès fluidique (P1>P2), l'objet biologique est piégé dans le deuxième piège hydrodynamique PH2 du dispositif de capture, selon le principe décrit ci-dessus ;

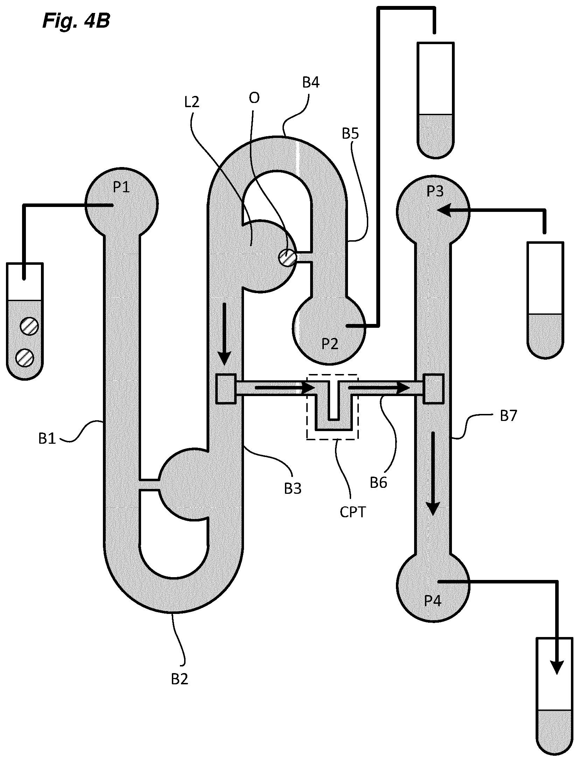

Figure 4B : Le premier accès fluidique A1 et le deuxième accès fluidique A2 sont mis à une même valeur de pression P1=P2. Le troisième accès fluidique A3 est placé à une valeur de pression supérieure à celle présente au niveau du quatrième accès fluidique A4 : P3>P4. La valeur de pression appliquée au niveau du premier accès fluidique A1 est également fixée supérieure à celle présente au niveau du troisième accès fluidique A3. Les sécrétions générées par l'objet biologique O traversent ainsi la branche fluidique de mesure B6 portant le capteur CPT et sont récupérées dans un réservoir connecté sur le quatrième accès fluidique A4.

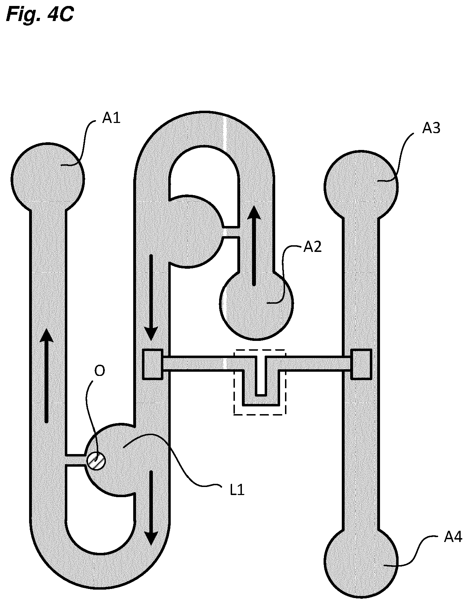

Figure 4C : En cas de modification du sens d'écoulement dans le premier circuit fluidique C1 (par exemple lié à un gradient de pression), l'objet biologique O peut être amené à se déloger du deuxième piège hydrodynamique PH2 pour rejoindre le premier piège hydrodynamique PH1, comme décrit ci-dessus en liaison avec les figures 2A à 2E. Le procédé d'analyse peut cependant se poursuivre sur le même objet biologique O, sans perte. Ce principe peut s'appliquer pour chaque accès fluidique, en adaptant les pressions exercées dans les circuits.

Les deux circuits fluidiques C1, C2 peuvent être réalisés dans un même composant micro-fluidique, par toutes techniques adaptées, par exemple par usinage et/ou micro-fabrication.

Les solutions présentées ci-dessus présentent de nombreux avantages, parmi lesquels :
- Le dispositif de capture garantit un maintien d'un objet biologique dans le circuit fluidique, même en cas de changement du sens d'écoulement dans ce circuit fluidique, consécutif par exemple à une déconnexion temporaire d'un des accès fluidiques pour permettre un changement de solution (par exemple un milieu de culture cellulaire ou encore des drogues, antiviraux, ou antibiotiques s'il s'agit d'une bactérie piégée) à laquelle sera exposé l'objet biologique ;
- Le système d'analyse utilisant un tel dispositif de capture permet d'analyser un objet biologique en continu, sans perte (voir point ci-dessus) ;
- La solution est simple à concevoir et facile à mettre en œuvre pour l'analyse d'un objet biologique ;

## Revendications

1. Dispositif de capture d'un objet biologique (O), comprenant un premier circuit fluidique (C1), ledit premier circuit fluidique (C1) comportant :
- Un premier accès fluidique (A1) et un deuxième accès fluidique (A2),
- Un canal principal s'étendant entre le premier accès fluidique et le deuxième accès fluidique, comprenant une première branche (B1) dans laquelle débouche le premier accès fluidique (A1), une branche centrale (B3) prolongeant ladite première branche, via une première branche de jonction (B2), et une deuxième branche (B5) prolongeant ladite branche centrale via une deuxième branche de jonction (B4) et débouchant sur le deuxième accès fluidique (A2),
- Un premier piège hydrodynamique (PH1) et un deuxième piège hydrodynamique (PH2),
- Chaque piège hydrodynamique étant réalisé sous la forme d'une branche de dérivation par rapport au canal principal et comportant un logement (L1, L2) dimensionné pour accueillir ledit objet biologique (O) et une restriction (R1, R2) prolongeant ledit logement,
- **Caractérisé en ce que** :
- La première branche (B1) et la deuxième branche (B5) du canal principal sont dénuées de logement appartenant à un piège hydrodynamique,
- Le premier piège hydrodynamique (PH1) est agencé de sorte que son logement (L1) communique d'un côté avec la branche centrale (B3) du canal principal et que sa restriction (R1) débouche de l'autre côté dans la première branche (B1) du canal principal,
- Le deuxième piège hydrodynamique (PH2) est agencé de sorte que son logement (L2) communique d'un côté avec la branche centrale (B3) du canal principal et que sa restriction (R2) débouche de l'autre côté dans la deuxième branche (B5) du canal principal.

2. Système d'analyse d'un objet biologique, comprenant un dispositif de capture dudit objet biologique et un dispositif de mesure, **caractérisé en ce que** :
- Le dispositif de capture est tel que défini en revendication 1, et **en ce que**
- Le dispositif de mesure comporte :
∘ Une branche fluidique de mesure (B6) connectée à une première extrémité à la branche centrale (B3) du dispositif de capture, entre ses deux pièges hydrodynamiques (PH1, PH2), et à une deuxième extrémité à un deuxième circuit fluidique (C2), distinct du premier circuit fluidique,
∘ Un capteur (CPT) coopérant avec la branche fluidique de mesure (B6).

3. Système selon la revendication 2, **caractérisé en ce que** le deuxième circuit fluidique (C2) comporte une branche fluidique de récupération (B7) s'étendant entre un troisième accès fluidique (A3) et un quatrième accès fluidique (A4) du deuxième circuit fluidique (C2), ladite deuxième extrémité de la branche fluidique de mesure (B6) étant connectée sur la branche fluidique de récupération (B7), entre le troisième accès fluidique (A3) et le quatrième accès fluidique (A4).

4. Système d'analyse selon la revendication 2 ou 3, **caractérisé en ce que** le capteur (CPT) est de type résonateur.

5. Système d'analyse selon la revendication 2 ou 3, **caractérisé en ce que** le capteur (CPT) est de type photonique.

6. Système d'analyse selon la revendication 2 ou 3, **caractérisé en ce que** le capteur (CPT) est configuré pour mesurer une grandeur électrique à travers la branche fluidique de mesure (B6).

7. Système selon l'une revendications 2 à 6, **caractérisé en ce que** le premier circuit fluidique (C1) et le deuxième circuit fluidique (C2) sont réalisés dans un composant fabriqué par micro-fabrication.

8. Procédé d'analyse d'un objet biologique, mis en œuvre à l'aide du système d'analyse tel que défini dans l'une des revendications 2 à 7, **caractérisé en ce qu'**il comporte :
- Une première étape d'injection de l'objet biologique (O) par le premier accès fluidique (A1) par application d'une première valeur de pression sur le premier accès fluidique (A1), supérieure à une deuxième valeur de pression présente sur le deuxième accès fluidique (A2),
- Piégeage hydrodynamique de l'objet biologique (O) dans le deuxième piège hydrodynamique (PH2),
- Application d'une troisième valeur de pression sur le premier accès fluidique (A1) et le deuxième accès fluidique (A2), et application d'une quatrième valeur de pression sur le troisième accès fluidique (A3) supérieure à une cinquième valeur de pression présente sur le quatrième accès fluidique (A4) et inférieure à ladite troisième valeur de pression,
- Récupération des sécrétions générés par ledit objet biologique (O) au niveau du quatrième accès fluidique (A4), après passage à travers la branche fluidique de mesure (B6)

## Patentansprüche

1. Vorrichtung zur Erfassung eines biologischen Objekts (O), die einen ersten Fluidkreislauf (C1) umfasst, wobei der erste Fluidkreislauf (C1) aufweist:
- Einen ersten Fluidzugang (A1) und einen zweiten Fluidzugang (A2),
- Einen Hauptkanal, der sich zwischen dem ersten Fluidzugang und dem zweiten Fluidzugang erstreckt, umfassend einen ersten Zweig (B1), in den der erste Fluidzugang (A1) mündet, einen zentralen Zweig (B3), der den ersten Zweig über einen ersten Verbindungszweig (B2) verlängert, und einen zweiten Zweig (B5), der den zentralen Zweig über einen zweiten Verbindungszweig (B4) verlängert und an dem zweiten Fluidzugang (A2) mündet,
- Eine erste hydrodynamische Falle (PH1) und eine zweite hydrodynamische Falle (PH2),
- Wobei jede hydrodynamische Falle in Form eines Bypasszweigs in Bezug auf den Hauptkanal ausgeführt ist und eine Aufnahme (L1, L2), die zum Aufnehmen des biologischen Objekts (O) dimensioniert ist, und eine die Aufnahme verlängernde Verengung (R1, R2) aufweist,
- **Dadurch gekennzeichnet, dass**:
- Der erste Zweig (B1) und der zweite Zweig (B5) des Hauptkanals keine Aufnahme aufweisen, die zu einer hydrodynamischen Falle gehört,
- Die erste hydrodynamische Falle (PH1) so angeordnet ist, dass ihre Aufnahme (L1) auf einer Seite mit dem zentralen Zweig (B3) des Hauptkanals kommuniziert und dass ihre Verengung (R1) auf der anderen Seite in den ersten Zweig (B1) des Hauptkanals mündet,
- Die zweite hydrodynamische Falle (PH2) so angeordnet ist, dass ihre Aufnahme (L2) auf einer Seite mit dem zentralen Zweig (B3) des Hauptkanals kommuniziert und dass ihre Verengung (R2) auf der anderen Seite in den zweiten Zweig (B5) des Hauptkanals mündet.

2. System zur Analyse eines biologischen Objekts, umfassend eine Vorrichtung zur Erfassung des biologischen Objekts und eine Messvorrichtung, **dadurch gekennzeichnet, dass**:
- Die Vorrichtung zur Erfassung wie in Anspruch 1 definiert ist, und dass
- Die Messvorrichtung aufweist:
∘ Einen Messfluidzweig (B6), der an einem ersten Ende an den zentralen Zweig (B3) der Vorrichtung zur Erfassung, zwischen ihren beiden hydrodynamischen Fallen (PH1, PH2), und an einem zweiten Ende an einen zweiten Fluidkreislauf (C2), der von dem ersten Fluidkreislauf verschieden ist, angeschlossen ist,
∘ Einen Sensor (CPT), der mit dem Messfluidzweig (B6) zusammenwirkt.

3. System nach Anspruch 2, **dadurch gekennzeichnet, dass** der zweite Fluidkreis (C2) einen Rückgewinnungsfluidzweig (B7) aufweist, der sich zwischen einem dritten Fluidzugang (A3) und einem vierten Fluidzugang (A4) des zweiten Fluidkreises (C2) erstreckt, wobei das zweite Ende des Messfluidzweigs (B6) an den Rückgewinnungsfluidzweig (B7) zwischen dem dritten Fluidzugang (A3) und dem vierten Fluidzugang (A4) angeschlossen ist.

4. System zur Analyse nach Anspruch 2 oder 3, **dadurch gekennzeichnet, dass** der Sensor (CPT) vom Resonatortyp ist.

5. System zur Analyse nach Anspruch 2 oder 3, **dadurch gekennzeichnet, dass** der Sensor (CPT) vom photonischen Typ ist.

6. System zur Analyse nach Anspruch 2 oder 3, **dadurch gekennzeichnet, dass** der Sensor (CPT) dazu ausgelegt ist, eine elektrische Größe durch den Messfluidzweig (B6) zu messen.

7. System nach einem der Ansprüche 2 bis 6, **dadurch gekennzeichnet, dass** der erste Fluidkreis (C1) und der zweite Fluidkreis (C2) in einer durch Mikrofertigung hergestellten Komponente ausgeführt sind.

8. Verfahren zur Analyse eines biologischen Objekts, das mithilfe des Systems zur Analyse wie in einem der Ansprüche 2 bis 7 definiert durchgeführt wird, **dadurch gekennzeichnet, dass** es umfasst:
- Einen ersten Schritt des Einleitens des biologischen Objekts (O) durch den ersten Fluidzugang (A1) durch Anwenden eines ersten Druckwerts auf den ersten Fluidzugang (A1), der größer als ein zweiter Druckwert ist, der an dem zweiten Fluidzugang (A2) vorliegt,
- Hydrodynamisches Einfangen des biologischen Objekts (O) in der zweiten hydrodynamischen Falle (PH2),
- Anwenden eines dritten Druckwerts auf den ersten Fluidzugang (A1) und den zweiten Fluidzugang (A2), und Anwenden eines vierten Druckwerts auf den dritten Fluidzugang (A3), der größer als ein fünfter Druckwert ist, der an dem vierten Fluidzugang (A4) vorliegt, und kleiner als der dritte Druckwert ist,
- Rückgewinnen der von dem biologischen Objekt (O) erzeugten Sekrete an dem vierten Fluidzugang (A4), nach dem Durchgang durch den Messfluidzweig (B6)

## Claims

1. Device for capturing a biological object (O), which device includes a first fluid circuit (C1), said first fluid circuit (C1) having:
- a first fluid port (A1) and a second fluid port (A2),
- a main channel extending between the first fluid port and the second fluid port, comprising a first branch (B1) into which the first fluid port (A1) opens, a central branch (B3) forming an extension of said first branch via a first junction branch (B2), and a second branch (B5) forming an extension of said central branch via a second junction branch (B4) and opening out at the second fluid port (A2),
- a first hydrodynamic trap (PH1) and a second hydrodynamic trap (PH2),
- each hydrodynamic trap taking the form of a branch from the main channel and including a housing (L1, L2) sized to accept said biological object (O) and a restriction (R1, R2) forming an extension of said housing,
- **characterized in that**:
- the first branch (B1) and the second branch (B5) of the main channel have no housing that is part of a hydrodynamic trap,
- the first hydrodynamic trap (PH1) is arranged so that its housing (L1) communicates on one side with the central branch (B3) of the main channel and its restriction (R1) opens on the other side into the first branch (B1) of the main channel, and
- the second hydrodynamic trap (PH2) is arranged so that its housing (L2) communicates on one side with the central branch (B3) of the main channel and its restriction (R2) opens on the other side into the second branch (B5) of the main channel.

2. System for analysis of a biological object, including a device for capturing said biological object and a measuring device, **characterized in that**:
- the capture device is as defined in Claim 1, and **in that**
- the measuring device includes:
∘ a measurement fluid branch (B6) connected at a first end to the central branch (B3) of the capture device between its two hydrodynamic traps (PH1, PH2) and at a second end to a second fluid circuit (C2) distinct from the first fluid circuit,
∘ a sensor (CPT) cooperating with the measurement fluid branch (B6).

3. System according to Claim 2, **characterized in that** the second fluid circuit (C2) includes a recovery fluid branch (B7) extending between a third fluid port (A3) and a fourth fluid port (A4) of the second fluid circuit (C2), said second end of the measurement fluid branch (B6) being connected to the recovery fluid branch (B7) between the third fluid port (A3) and the fourth fluid port (A4).

4. Analysis system according to Claim 2 or 3, **characterized in that** the sensor (CPT) is of resonator type.

5. Analysis system according to Claim 2 or 3, **characterized in that** the sensor (CPT) is of photonic type.

6. Analysis system according to Claim 2 or 3, **characterized in that** the sensor (CPT) is configured to measure an electrical parameter in the measurement fluid branch (B6).

7. System according to one of Claims 2 to 6, **characterized in that** the first fluid circuit (C1) and the second fluid circuit (C2) are produced in a component manufactured by micro-manufacture.

8. Method for analysing a biological object using the analysis system as defined in any of Claims 2 to 7, **characterized in that** it includes:
- a first step of injection of the biological object (O) via the first fluid port (A1) by application of a first pressure at the first fluid port (A1) greater than a second pressure at the second fluid port (A2),
- hydrodynamic trapping of the biological object (O) in the second hydrodynamic trap (PH2),
- application of a third pressure to the first fluid port (A1) and the second fluid port (A2) and application of a fourth pressure to the third fluid port (A3) greater than a fifth pressure at the fourth fluid port (A4) and less than said third pressure,
- recovery of the secretions generated by said biological object (O) at the level of the fourth fluid port (A4) after passage through the measurement fluid branch (B6).
